# EUROPEAN PATENT APPLICATION

(11) **EP 3 878 966 A1**
(43) Date of publication of application: **15.09.2021**
(21) Application number: 19881064.0
(22) Date of filing: 07.11.2019
(51) Int. Cl.: C12P 19/02, A23L 27/00, C12N 9/90, C12N 9/92

(54) **METHOD FOR PRODUCING RARE SUGAR-CONTAINING COMPOSITION AND RARE SUGAR-CONTAINING COMPOSITION**

(30) Priority: 08.11.2018 JP 2018210408
(71) Applicant: National University Corporation Kagawa University, Takamatsu-shi, Kagawa 760-8521 (JP)
(72) Inventor: AKIMITSU, Kazuya, Takamatsu-shi, Kagawa 760-8521 (JP); IZUMORI, Ken, Takamatsu-shi, Kagawa 760-8521 (JP); TAKAOKA, Seizo, Takamatsu-shi, Kagawa 760-8521 (JP); YOSHIHARA, Akihide, Takamatsu-shi, Kagawa 760-8521 (JP); KATO, Shiro, Takamatsu-shi, Kagawa 760-8521 (JP); MOCHIZUKI, Susumu, Takamatsu-shi, Kagawa 760-8521 (JP)
(74) Representative: TBK
(86) International application number: PCT/JP2019/043722
(87) International publication number: WO 2020/096006

(57) **Abstract**

An object of the present invention is to provide a rare sugar-containing composition having improved properties as a sweetener while maintaining the excellent functionality of D-psicose. The invention relates to a method of producing a rare-sugar containing composition with D-fructose as a raw material. An intended product having a novel composition of D-glucose, D-fructose, and D-psicose is obtained by preparing a mixture of D-fructose and D-psicose by using ketose 3-epimerase in the first stage and converting D-fructose into D-glucose by using D-xylose isomerase inert to D-psicose in the second stage. The first-stage product is a mixture of D-fructose and rare sugar D-psicose. The resulting mixture is a sweetener having a taste quality equal to that of sugar.

## Description

### TECHNICAL FIELD

The present invention relates to a method of producing a sugar-containing composition capable of producing 100% of raw materials as a product in principle without losing them due to a separation operation or the like during its production step by using D-fructose as a raw material, performing two enzymatic reactions, and using these two enzymatic reactions in combination in series; and a rare sugar-containing composition obtained as a product and having a taste quality equal to that of sugar and uses of the composition.

Here, ketose 3-epimerase and xylose isomerase which are enzymes to be used in the present invention are defined.

### (1) Isomerase

Isomerase catalyzes "a reaction not causing a change in chemical formula". Isomerase which acts on hexose catalyzes "a reaction not causing a change in chemical formula C₆H₁₂O₆". For example, aldose isomerase catalyzes "an intramolecular hydrogen transfer" and accelerates a redox reaction between C₁ and C₂.

### (2) Epimerase

Epimerase is an enzyme which transfers, when used for a monosaccharide, the position of -OH bound to C to the reverse side and catalyzes "a reaction not causing a change in chemical formula C₆H₁₂O₆". It is therefore an enzyme belonging to isomerase.

### (3) Conclusion

Isomerase is superordinate to epimerase. Described specifically, an enzyme "isomerase" is well known in this industry and examples of it include ketose 3-epimerase, xylose isomerase, and glucose isomerase. Glucose isomerase is however not a scientifically acknowledged name. The enzyme name "glucose isomerase" is not included in EC number registered in International Union of Biochemistry and Molecular Biology. It is not "a scientific enzyme name". D-xylose isomerase is its scientific name and glucose isomerase is only used in general as a commercial name. The reason is that "humans only make use of' the action of D-xylose isomerase on glucose having a similar structure and in the natural world, glucose is not used after converted into fructose.

Epimerase is included in isomerase. It can therefore be expressed as (a) "an enzyme that isomerizes D-glucose into D-fructose" or (b) "an enzyme that isomerizes D-fructose into D-psicose".

For example, even if L-rhamnose isomerase of a certain microorganism acts on D-glucose and converts it into D-fructose, the above-described (a) "an enzyme that isomerizes D-glucose into D-fructose" also includes this L-rhamnose isomerase. It includes all the proteins that are possessed by any organisms such as microorganism, vegetable, and animal and convert D-glucose into D-fructose.

The above-described (b) "an enzyme that isomerizes D-fructose into D-psicose" also includes proteins produced by any organisms when it converts D-fructose into D-psicose.

All the enzymes belonging to "EC.5. ― (Isomerase)" in EC number are included. This means that even if a newly discovered enzyme or an enzyme classified as another isomerase has action capable of catalyzing the above reactions, though only slightly is included in it. The name "isomerase" as used in the present invention means a wide enzyme group.

### BACKGROUND ART

In the conventional art, pure rare sugar D-allulose (D-psicose) is produced using D-fructose as a raw material in the presence of ketose 3-epimerase (for example, D-allulose 3-epimerase). The rare sugar D-psicose can be produced as follows. First, epimerase is allowed to act on D-fructose to prepare a mixture of D-fructose and D-psicose. Then, the mixture is separated and purified to obtain D-psicose as a crystalline powder. This D-psicose itself is used as a sweetener, pharmaceutical, or the like so that mass production and sales of it have been started in the world. Since this D-psicose is a zero energy material and has various functions, sales of it particularly as a sweetener has proceeded.

Rare Sugar Sweet (RSS), a rare sugar-containing syrup has been produced by a chemical reaction using a fructose-glucose syrup as a raw material (refer to Patent Document 5).

The sweetness of D-psicose as a sweetener is about 70% of that of sugar so that the sweetness intensity of it should be brought closer to that of sugar.

According to Patent Document 1, a sweetener has been developed with a view to overcoming the problem of a fructose-glucose syrup as a conventional sweetener, that is, a difference in sweetness and taste quality from sugar and preparing a sweetener having a sweetness intensity and taste quality very close to those of sugar. The sweetener is composed of D-fructose, D-glucose, and D-psicose, has a sweetness intensity and a taste quality almost similar to those of sugar, and controls the obesity effect of a fructose-glucose syrup or high fructose corn syrup. More specifically, it is composed of 9 parts by weight or more of D-psicose based on 100 parts by weight in total of D-glucose and D-psicose, from 34 to 55 parts by weight of D-fructose based on 100 parts by weight in total of D-fructose, D-glucose, and D-psicose, and from 66 to 45 parts by weight in total of D-glucose and D-psicose based on 100 parts by weight in total of D-fructose, D-glucose, and D-psicose.

As a method of producing the above-described sweetener capable of producing even a D-psicose-containing sweetener at a low cost, shown are following methods using a so-called fructose-glucose syrup as a raw material: (1) a method using a continuous plant, (2) a method using a mixed enzyme, and (3) a method of preparing a high D-psicose-containing sugar solution.

### (1) Method using a continuous plant

First, a fructose-glucose syrup is prepared and then, ketose 3-epimerase is allowed to act thereon. The fructose-glucose syrup is prepared by using a starch as a raw material and using an enzyme such as α-amylase, glucoamylase, or glucose isomerase in immobilized or batch system. Then, epimerase is continuously allowed to act on the fructose-glucose syrup solution thus obtained to prepare a mixed sugar solution of D-glucose, D-fructose, and D-psicose. According to Examples, the sugar solution thus obtained is composed of 58 parts by weight of D-glucose, 34 parts by weight of D-fructose, and 8 parts by weight of D-psicose. The D-psicose content of the product is about 8% and thus relatively low.

### (2) Method using a mixed enzyme

Glucose isomerase + ketose 3-epimerase is allowed to act on the above-described glucose syrup. An immobilized enzyme containing isomerase and epimerase is packed in an appropriate column, the degraded glucose syrup is continuously poured in the column, and a reaction product is collected.

According to Examples, a sugar solution obtained after a 2-hour reaction is composed of 41 parts by weight of D-glucose, 48 parts by weight of D-fructose, and 11 parts by weight of D-psicose. A sugar solution obtained after a 1-hour reaction is composed of 43 parts by weight of D-glucose, 48 parts by weight of D-fructose, and 9 parts by weight of D-psicose.

### (3) Method of preparing a high D-psicose-containing sugar solution

An immobilized mixed enzyme of glucose isomerase and D-tagatose 3-epimerase is added to a glucose syrup to cause a reaction there between. According to Examples, the sugar solution obtained after a 4-hour reaction is composed of 54 parts by weight of D-glucose, 36 parts by weight of D-fructose, and 10 parts by weight of D-psicose and the syrup obtained after a 20-hour reaction is composed of 41 parts by weight of D-glucose, 42 parts by weight of D-fructose, and 17 parts by weight of D-psicose.

Another effort has been made to provide - like a D-psicose-containing low-calorie sweetener as described in Patent Document 2 containing D-psicose as a main ingredient, containing a sugar alcohol and/or a sweetener with high sweetness intensity, and having an improved taste quality - a sweetener having an improved sweetness intensity while maintaining its function by the addition of a sweetener with high sweetness intensity or addition of another sweetener.

There are many problems to be overcome in the industrial mass production of these rare sugar-containing compositions.

For example, when D-ketohexose 3-epimerase (Patent Document 3) is allowed to act on D-fructose, rare sugar D-psicose is produced in a yield of from 20 to 25% from D-fructose. According to the report, when D-psicose 3-epimerase (Non-Patent Document 1) is used, on the other hand, D-psicose is produced in a yield of 40%, while when boric acid is used in combination, D-psicose is produced in a yield of 62%. When production of purified D-fructose is followed by production of D-psicose, they are performed in respective steps so that it is really very difficult to achieve industrial mass production of D-psicose due to limitations in raw materials, transport, reaction cost, plant management cost, and the like.

There is a high-fructose corn syrup having D-glucose and D-fructose at a ratio of about 58:42 and this fructose-glucose syrup (58% high-fructose corn syrup, HFCS) is a product still in use today as a sweetener. For it, a composition having D-glucose and D-fructose at a ratio in the equilibrium of about 58:42 in the procedure of converting D-glucose into D-fructose in the presence of isomerase and obtained in the production procedure without separating the ingredients is used as is. Since the concentration of D-fructose having a high sweetness intensity in the product is low so that it is used as HFCS after D-fructose is separated and added to a product. Thus, the composition obtained in the production procedure without separating the ingredients is never used as is as a sweetener.

For the preparation of a fructose-glucose syrup from a starch, three enzymatic reactions and purification and concentration are required, which will be described in detail below.
(1) Liquefaction: To a starch are added water and α-amylase as a hydrolase, and the resulting mixture is heated to about 95°C. This degrades the high-molecular-weight starch to make it smaller to some extent.
(2) Saccharification: After completion of the liquefaction, the resulting liquid is cooled to about 55°C, followed by the addition of glucoamylase. This reaction degrades the sugar to make it further smaller and thus, glucose is obtained.
(3) Isomerization: About half of the glucose is converted into fructose by adding glucose isomerase as an isomerized enzyme at 60°C. The name "fructose-glucose syrup" is derived from this reaction (reaction of isomerizing glucose to fructose).
(4) Purification/Concentration: After isomerization, the syrup is purified using a filtering device or ion exchanger and then concentrated by evaporation of water to obtain a fructose-glucose syrup having a fructose content of 42%. The fructose purify is then increased by chromatography, by which a high fructose syrup having a fructose content of from 90 to 95% can be obtained. The resulting syrup is blended with a fructose-glucose syrup having a fructose content of 42% to prepare a fructose glucose solution having a fructose content of 55%.

Considering that the sweetener described in Patent Document 1 has a sweetness intensity and a taste quality almost similar to those of sugar and has a function of suppressing an obesity effect of a fructose-glucose syrup or high fructose corn syrup, it is time for such a sweetener to appear in the market. There is therefore an urgent demand for the improvement of a production method to achieve a cost reduction or the like and development of a mass production technique, based on the method capable of producing even a D-psicose-containing sweetener at a low cost which was also an object of the invention.

### Prior Art Documents

### Patent Documents

Patent Document 1: Japanese Patent No. 5639759
Patent Document 2: Japanese Patent No. 4942001
Patent Document 3: Japanese Patent Application Laid-Open No. He 6-125776
Patent Document 4: Japanese Patent No. 5922880
Patent Document 5: Japanese Patent No 5715046
Patent Document 6: Japanese Patent No. 5997693
Patent Document 7: Japanese Patent Laid-Open No. 2017-36276
Patent Document 8: Japanese Patent No. 4009720
Patent Document 9: Japanese Patent Application Laid-Open No. 2007-91696

### Non-Patent Document

Non-Patent Document 1: The 3rd Symposium of International Society of Rare Sugars

### SUMMARY

### Technical Problem

An object of the present invention is to provide a rare sugar-containing composition having improved properties as a sweetener while maintaining the excellent functionality of D-psicose.

The reason why a fructose-glucose syrup or high fructose corn syrup is said to be a main cause of obesity is because D-fructose (fructose) has an adverse effect on the accumulation of neutral fat. D-fructose has, on the other hand, an advantage of its sweet and delicious taste. What is requested is to adjust the amount of D-fructose to prevent appearance of its defect and adjust the composition to bring out the advantage of D-fructose that it is sweet; to make up for the insufficient sweetness intensity which is a disadvantage of D-psicose; and to create a new sweetener having the functionality of D-psicose.

Although it is the common practice to improve the sweetness intensity of D-psicose by adding a sweetener with high sweetness intensity or another sweetener, an object of the present invention is to provide a method of producing a rare sugar-containing composition in a convenient manner without adding anything by using a raw material and two enzymatic reactions and skillfully using these reactions in combination.

Another object is to provide a production method characterized in that production is achieved by reacting epimerase and isomerase under respectively suitable conditions without spending an additional equipment investment cost and can be performed by freely using a continuous reaction or individual reactions.

A further object is to provide a production method completed by constructing a unique system capable of improving the sweetness intensity and taste quality to be equal to those of sugar by adjusting a D-fructose content, capable of reducing a production cost by connecting its line to a conventional production line in an appropriate manner, and capable of maintaining the function of D-psicose sufficiently.

### Solution to Problem

The present inventors have pursued extensive investigation with a view to overcoming the above-described problems. During the investigation, it has been found that a sweetener having a sweetness intensity and a sweetness quality equal to those of sugar and moreover, effective for preventing life style related diseases such as obesity can be obtained from a rare sugar-containing composition composed of D-glucose, D-fructose, and D-psicose at a certain ratio; and that when a conventional production line is connected in an appropriate manner, the rare sugar-containing composition can be produced in a pure form not containing a side-reaction product and addition of a special purification step is not necessary, leading to the completion of the present invention.

The method is completed as that for obtaining a product having a composition close to an intended one by adjusting the activity of an enzyme and flow rate of an immobilized enzyme column to be used in this method.

The present invention has, as a gist thereof, the following methods (1) to (13) of producing a rare sugar-containing composition.
(1) A method of producing a rare sugar-containing composition with D-fructose as a raw material, including a first stage of preparing a mixture of D-fructose and D-psicose by using ketose 3-epimerase and a second stage of converting D-fructose into D-glucose by using D-xylose isomerase inert to D-psicose to obtain an intended product having a new composition of D-glucose, D-fructose, and D-psicose.
(2) The method as described above in (1), wherein the first-stage product is a mixture of D-fructose and rare sugar D-psicose.
(3) The method as described above in (1), wherein the first-stage product is an equilibrium mixture of D-fructose and rare sugar D-psicose.
(4) The method as described above in (1), wherein the first-stage product is a mixture of D-fructose and rare sugar D-psicose adjusted to have an intended concentration.
(5) The method as described above in any of (1) to (4), wherein in the second stage, D-xylose isomerase is continuously or individually allowed to act on the first-stage product.
(6) The method as described above in any of (1) to (5), wherein the second-stage intended product is a composition having from 10 to 20 parts by weight of rare sugar D-psicose and from 90 to 80 parts by weight of D-glucose and D-fructose, each based on 100 parts by weight in total of rare sugar D-psicose, D-glucose, and D-fructose.
(7) The method as described above in any of (1) to (5), wherein the second-stage intended product is a composition having from 15 to 20 parts by weight of rare sugar D-psicose and from 85 to 80 parts by weight of D-glucose and D-fructose, each based on 100 parts by weight in total of rare sugar D-psicose, D-glucose, and D-fructose.
(8) The method as described above in any of (1) to (7), wherein the second-stage intended product is a rare sugar-containing composition having a taste quality equal to that of sugar.
(9) The method as described above in any of (1) to (8), wherein the intended product having an intended composition is obtained by adjusting the enzymatic activity of ketose 3-epimerase and D-xylose isomerase to be used in this method.
(10) The method as described above in any of (1) to (9), wherein the reaction in the first stage and the reaction in the second stage are performed by passing substrate solutions through immobilized enzyme columns, respectively, and an intended product having an intended composition is obtained by adjusting a flow rate of substrates.
(11) The method as described above in any of above (1) to (10), wherein an immobilized enzyme reaction is performed with raw material D-fructose as a substrate and a 100% intended product is obtained theoretically without producing a by-product.
(12) The method as described above in any of (1) to (11), wherein the intended product is not lost during a production step by a separating operation using simulated moving bed chromatography.
(13) The method as described above in any of (1) to (12), wherein a saccharide in crystal form or solid form is produced from a syrup itself obtained by passing the second-stage intended product through an immobilized enzyme column.

The present invention has, as a gist thereof, rare sugar-containing compositions described below in (14) to (19) having a taste quality equal to that of sugar and the use of the compositions described below in (20) to (29).
(14) A rare sugar-containing composition having a taste quality equal to that of sugar, having from 10 to 20 parts by weight of rare sugar psicose and from 90 to 80 parts by weight of D-glucose and D-fructose, each based on 100 parts by weight in total of rare sugar D-psicose, D-glucose, and D-fructose.
(15) A rare sugar-containing composition having a taste quality equal to that of sugar, having from 15 to 20 parts by weight of rare sugar psicose and from 85 to 80 parts by weight of D-glucose and D-fructose, each based on 100 parts by weight in total of rare sugar D-psicose, D-glucose, and D-fructose.
(16) The rare sugar-containing composition having a taste quality equal to that of sugar as described above in (14) or (15), which is a product of raw material D-fructose and at the same time, a product not subjected to a separating operation by simulated moving bed chromatography.
(17) The rare sugar-containing composition having a taste quality equal to that of sugar as described above in (14), (15), or (16), which is a product of raw material D-fructose and at the same time, is a syrup itself obtained by passing through the immobilized enzyme reaction column.
(18) The rare sugar-containing composition having a taste quality equal to that of sugar as described above in from (14) to (17), which is a sweetener having a taste quality equal to that of sugar.
(19) The rare sugar-containing composition having a taste quality equal to that of sugar as described above in from (14) to (18), which is in syrup form or in crystalline form.
(20) A sugar-like sweetener containing the rare sugar-containing composition having a taste quality equal to that of sugar as described above in any of (14) to (19).
(21) A food containing the rare sugar-containing composition having a taste quality equal to that of sugar as described above in any of (14) to (19).
(22) A pharmaceutical or quasi drug containing the rare sugar-containing composition having a taste quality equal to that of sugar as described above in any of (14) to (19).
(23) An oral composition containing the rare sugar-containing composition having a taste quality equal to that of sugar as described above in any of (14) to (19).
(24) A cosmetic containing the rare sugar-containing composition having a taste quality equal to that of sugar as described above in any of (14) to (19).
(25) An anti-obesity agent containing the rare sugar-containing composition having a taste quality equal to that of sugar as described above in any of (14) to (19).
(26) An appetite suppressant containing the rare sugar-containing composition having a taste quality equal to that of sugar as described above in any of (14) to (19).
(27) An insulin-resistance improver containing the rare sugar-containing composition having a taste quality equal to that of sugar as described above in any of (14) to (19).
(28) A low-calorie sweetening agent containing the rare sugar-containing composition having a taste quality equal to that of sugar as described above in any of (14) to (19).
(29) A sugar-like sweetening agent containing the rare sugar-containing composition having a taste quality equal to that of sugar as described above in any of (14) to (19).

### Advantageous Effects of Invention

The present invention can provide a rare sugar-containing composition having improved properties as a sweetener while maintaining the excellent functionality of D-psicose. In spite that D-fructose (fructose) has such an advantage that it is sweet and delicious, it is disadvantageous because of having an adverse effect on the accumulation of neutral fat. A fructose-glucose syrup containing it is therefore regarded as a main cause for obesity. It is possible to create a new sweetener containing D-fructose in an amount adjusted to prevent appearance of the disadvantage, having a composition adjusted to bring out the advantage of D-fructose in sweetness, making up for insufficient sweetness which is a weak point of D-psicose, and having functionality of D-psicose. Since the rare sugar-containing composition having a taste quality equal to that of sugar according to the present invention is a new composition composed of D-glucose, D-fructose, and D-psicose, it can be used not only for food and beverages, particularly functional foods (for obesity prevention, or the like) but also for pharmaceuticals, cosmetics, feed, agricultural chemicals (plant growth regulators, plant defense elicitor, and the like), and industrial uses.

The present invention can provide a method of producing a rare sugar-containing composition in a convenient manner without adding anything by using raw materials and two enzymatic reactions and skillfully using these two reactions in combination. The present invention can provide a production method characterized in that an additional equipment investment cost is not required, production is completed by reacting epimerase and isomerase under respective conditions suited thereto, and production can be performed by freely using a continuous reaction or individual reaction.

When conventional saccharide sweeteners such as HFCS or D-psicose are produced, an enzymatic reaction is an equilibrium reaction so that a separating operation by simulated moving bed chromatography is essential for the production of an intended sweetener. In the present invention, all the raw material D-fructose can be converted into a product in a yield of 100% theoretically without using this separating operation.

The present invention can provide a production method completed by constructing a unique system capable of improving the sweetness intensity to be equal to that of sugar by adjusting a D-fructose content, reducing a production cost by connecting to a conventional production line in an appropriate manner, and maintaining the function of D-psicose sufficiently.

Since the separating operation is omitted, a decrease in concentration due to separation hardly occurs, a concentration operation can be omitted, and a product can be obtained only by a deionizing operation and concentration of a product of the enzymatic reaction. A drastic cost reduction can therefore be expected.

### BRIEF DESCRIPTION OF DRAWINGS

Fig. 1 is a diagram showing a step of producing a novel rare sugar-containing composition of the present invention.
Fig. 2 shows the results of analyzing the novel rare sugar-containing composition of the present invention by high-performance liquid chromatography (HPLC).
Fig. 3 shows the method of producing a novel sweetener (Patent Document 1) having a sugar-like taste quality and a production method of the present invention in contrast.
Fig. 4 is a diagram for describing the taste quality of an intended product of the present invention as a sweetener.
Fig. 5 comparatively shows, for describing the properties of the novel rare sugar-containing composition of the present invention, commercially available RSS (Patent Document 4), the novel sweetener having a sugar-like taste quality (Patent Document 1), and a simple addition method.

### DESCRIPTION OF EMBODIMENTS

The present invention relates to a method of producing a composition having an intended new composition composed of rare sugar D-psicose, D-glucose, and D-fructose by allowing ketose 3-epimerase to act on raw material D-fructose to prepare a mixture of D-fructose and rare sugar D-psicose and allowing D-xylose isomerase inert to D-psicose to act on the resulting mixture to convert D-fructose into D-glucose; and the resulting rare sugar-containing composition having a taste quality equal to that of sugar and uses of the composition.

First, the production method will be described.

### [Production method]

It is a method of producing a rare sugar-containing composition using D-fructose as a raw material and is a method characterized in that an intended product having a new composition composed of D-glucose, D-fructose, and D-psicose is obtained by allowing ketose 3-epimerase to act on D-fructose to obtain a mixture of it with D-psicose in the first stage and allowing D-xylose isomerase inert to D-psicose to act on D-fructose to convert it into D-glucose in the second stage.

The production method of the present invention will be described in further detail.

### (1) Using D-fructose as a raw material

D-fructose has conventionally been used as a raw material also for preparation of D-psicose. RSS is a fructose-glucose syrup but uses D-fructose as a raw material. The present invention is characterized in that D-fructose is used as a raw material for producing a mixture of D-glucose, D-fructose, and D-psicose.

(2) Production of rare sugar D-psicose by allowing ketose 3-epimerase to act on the raw material in the first reaction in the first stage. In the first reaction, a mixture of D-fructose and rare sugar D-psicose is obtained. The mixture of D-fructose and rare sugar D-psicose is an equilibrium mixture of D-fructose and rare sugar D-psicose or a mixture of D-fructose and rare sugar D-psicose having a concentration adjusted to an intended one. The composition of D-psicose can be changed by controlling the reaction so as not to reach equilibrium.

### (3) Allowing isomerase to act on the first-stage product in the second stage

Since D-xylose isomerase is inert to D-psicose, only D-fructose in the first-stage product is converted into D-glucose to obtain a mixture of D-glucose, D-fructose, and D-psicose. An intended product having a new composition is thus produced.

(4) The second-stage intended product is a rare sugar-containing composition having a taste quality equal to that of sugar.

Created is a new sweetener having D-fructose in an amount adjusted so as to prevent appearance of an adverse effect on the accumulation of neutral fat or the like, having a composition adjusted to bring out an advantage of D-fructose in sweetness, capable of making up for an insufficient sweetness intensity which is a weak point of D-psicose, and having functionality of D-psicose. By expressing the sweetness intensity of the product as a calculated value based on the actual results, it can be evaluated as a sweetener having a taste quality equal to that of sugar.

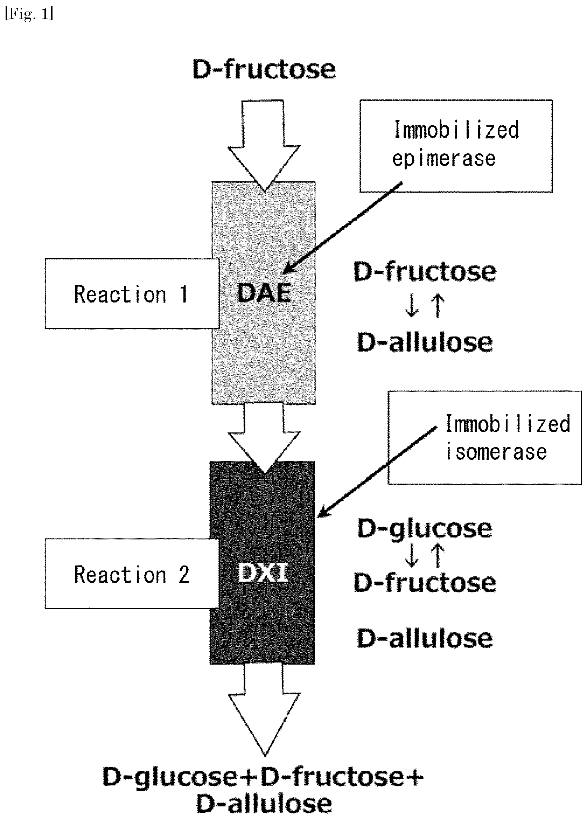

The production method of the present invention will be shown schematically in Fig. 1.

By passing the raw material D-fructose through two bioreactors (immobilized epimerase column [Reaction 1] and immobilized isomerase column [Reaction 2]), a reaction proceeds in order and an intended product having a new composition composed of D-glucose, D-fructose, and D-psicose is obtained.

### [Reaction 1]

The reaction in the immobilized epimerase column will be described.

The raw material fructose reacts with epimerase and an about 4:1 mixture of fructose and psicose comes out from the column of Reaction 1.

### [Reaction 2]

The reaction product mixture obtained in Reaction 1, that is, the about 4:1 mixture of D-fructose and D-psicose is introduced into the immobilized isomerase column of Reaction 2.

In the reaction of Reaction 2, isomerase acts on D-fructose of the D-fructose and D-psicose mixture introduced from Reaction 1 but is inert to D-psicose.

### [Products of Reactions 1 and 2]

The reaction in the immobilized isomerase column will next be described.

In Reaction 1, D-fructose is converted into D-psicose and a mixture of D-fructose and D-psicose is produced. If necessary, the reaction can be continued until it reaches equilibrium. The mixture is introduced into the column of Reaction 2 and in Reaction 2, isomerase reacts only with D-fructose and a mixture of D-glucose, D-fructose, and D-psicose is produced. If necessary, the reaction can be continued until it reaches equilibrium. When it reaches equilibrium, a rare sugar-containing composition having D-glucose, D-fructose, and D-psicose at a ratio of about 44:36:20 is obtained. This rare sugar-containing composition having this composition ratio is a novel composition. This means that the rare sugar-containing composition thus obtained is a rare sugar-containing composition (sweetener) having sweet and delicious D-fructose in an amount so as to prevent appearance of its disadvantage (adverse effect on accumulation of neutral fat or the like) but make up for the insufficient sweetness intensity which is a weak point of D-psicose, containing D-psicose in an amount enough to exhibit the functionality of D-psicose, and therefore having a taste quality equal to that of sugar.

### [Characteristics and advantages of production method]

### [Characteristic 1]

The reactions of epimerase and isomerase can be made in separately-placed immobilized reaction columns so that an intended reaction can be allowed to proceed readily under appropriate conditions.

### [Characteristic 2]

The reactions of these two enzymes can be allowed to proceed under proper reaction conditions, respectively. This makes it possible to conveniently respond to occurrence of activity reduction or the like by exchanging the corresponding column.

### [Characteristic 3]

Since two enzymatic reactions are performed separately, a reaction of only an intended sugar can be performed. A large characteristic is that an intended product having a sweetness intensity close to that of sugar can be obtained by skillfully controlling the reactions, depending on the specificity of these two reactions. Created is a sweetener containing D-fructose in an amount not causing too much sweetness but enough for making up for the insufficient sweetness of D-psicose which is a disadvantage thereof, having the functionality of D-psicose, and therefore having a sweetness balance among D-glucose, D-fructose, and D-psicose close to that of sugar.

### [Advantage 1]

As a manufacturing apparatus necessary for the production of this novel rare sugar-containing composition, an apparatus obtained only by connecting, as is, an isomerase column which has been used generally for the production of a fructose-glucose syrup to an apparatus for producing D-psicose from D-fructose can be used. This enables very low-cost production.

### [Advantage 2]

The composition of the novel rare sugar-containing composition can easily be made constant or changed by controlling the enzymatic reaction of epimerase or isomerase.

### [Advantage 3]

Since the production step does not include a separation procedure, the production step is can be made simpler. This leads to a drastic cost reduction and in addition, enables easy management of the production step.

### [Advantage 4]

During production, the raw material or product is not lost by the separating operation or the like in the production step and 100% of all the materials can be obtained as a product in principle.

### [Advantage 5]

A composition ratio of the product can be changed by adjusting the two enzymatic activities or adjusting the passage rate through a reaction column.

### [Isomerase]

Ketohexose 3-epimerase to be used for the production of the intended product of the present invention having a new composition composed of D-glucose, D-fructose, and D-psicose may have any enzyme origin or classification name insofar as it is an enzyme that isomerizes D-fructose into D-psicose [for example, bacteria belonging to the genus Pseudomonas described in Patent Document 1, D-ketohexose 3-epimerase available from Arthrobacter globiformis M30 strain (International Depositary Number NITE BP-1111) described in Patent Documents 6, and Non-Patent Document 1].

No limitation is imposed on the origin or nomenclature of the enzyme D-xylose isomerase insofar as it is an enzyme having ability of isomerizing D-glucose into D-fructose (for example, Patent Documents 1 and 4).

These enzymes may be purified enzymes or microorganisms producing these enzymes. In the present invention, the purified enzymes or enzyme-producing microorganisms are used as immobilized enzymes or immobilized microorganisms having the purified enzymes or the enzyme-producing microorganisms immobilized thereon, respectively.

### [Immobilization of enzyme]

It is most useful to immobilize an enzyme on an appropriate base material such as ion exchange resin. To the whole production step, either a batch process or continuous process may be applied. When a continuous process is employed, an epimerization reaction step of raw material D-fructose and an isomerization step of a mixture of the reaction product D-fructose and D-psicose may be performed as a series of steps and further, production is carried out while immobilizing D-tagatose 3-epimerase and glucose isomerase, respectively. Such a production method of performing an epimerization reaction step with D-fructose as a raw material and an isomerization step of the reaction product as a series of steps and further, using immobilized enzymes respectively for the production is a novel production method which cannot be found before.

For epimerization with an enzyme and isomerization with another enzyme, the enzymes are used in immobilized form and immobilized enzymes which are stable and easily usable can be obtained by various immobilization method. Using immobilized enzymes enables a large amount of epimerization and isomerization reactions continuously. For example, the reactions are made using immobilized enzymes each having an activity of 1000 U/wet weight resin (g). An immobilized enzyme can be obtained by collecting a crude enzyme solution from a solution of disrupted cells obtained by disrupting fungus body cells, passing the crude enzyme solution through an ion exchange resin packed in a column at a low temperature (4°C) to bind the crude enzyme protein to the ion exchange resin, and passing purified water for washing. An immobilization system capable of withstanding continuous production from the standpoint of stability (maintenance of activity) completely satisfactory for commercial production is obtained. A large amount of an epimerization reaction (epimerization reaction) can be performed continuously with the immobilized enzyme thus obtained.

### [Consideration on using an equilibrium-state composition as is as a sweetener]

By allowing D-ketohexose 3-epimerase (Patent Document 3) to act on D-fructose, rare sugar D-psicose is prepared from D-fructose in a yield of from 20 to 25%. An object of the method described in Patent Document 3 is to produce D-psicose so that an equilibrium-state composition after the reaction obtained using D-fructose as a starting material is for the separation of D-psicose from the composition. The equilibrium-state composition is not an intended composition but is an intermediate composition from which D-psicose is separated.

Using the equilibrium-state composition as a sweetener has many problems to overcome such as whether it is used in a syrup form as is after purification, or whether it is used as a saccharide in a crystal form or a solid form.

For production of a conventional saccharide sweetener such as HFCS or D-psicose, a separating operation by simulated moving bed chromatography is indispensable to produce an intended sweetener because an enzymatic reaction is an equilibrium reaction. In the present invention, without performing this separating operation, a product can be produced from the whole portion of raw material D-fructose, that is, in a yield of 100% theoretically. Since the composition of the present invention has a new composition composed of D-glucose, D-fructose, and D-psicose, the technique described in Patent Document 4 for conveniently producing a saccharide in crystal form or solid form from a syrup mainly containing D-glucose, D-fructose, and the like can be applied.

As a technique making use of an equilibrium-state composition as a sweetener, there is a technique of a fructose-glucose syrup. When a fructose-glucose syrup is industrially obtained, a fructose-glucose syrup having a fructose concentration less than 50%, a fructose-glucose syrup having a fructose concentration of 50% or more and less than 90%, a high fructose syrup having a fructose concentration of 90% or more, or the like is produced by adding glucose isomerase, which is an isomerase, to a glucose solution, reacting the resulting mixture at about 60°C to convert a portion of glucose to fructose, and then increasing a fructose purity by transpiring water, concentrating, and performing separation and purification by chromatography. A fructose-glucose syrup, which is a mixed sugar solution of glucose (D-glucose) and fructose (D-fructose), is used in liquid form due to great difficulty in crystallizing it. This is because in general, it is very difficult to convert a liquid sugar containing a plurality of sugars into a crystal form and there remains no way but using it in a liquid form.

Under such situations, the rare sugar-containing fructose-glucose syrup described in Patent Document 4 contains, in addition to D-glucose and D-fructose, a plurality of rare sugars such as D-psicose and D-allose so that is it obvious from "a pure single material crystallizes easily and a material containing an impurity is hard to crystallize" that the fructose-glucose syrup is a sparingly crystallizable composition.. The invention overcomes such a problem of the fructose-glucose syrup and enables production of a saccharide in crystal form or solid form from a saccharide mainly containing D-glucose, D-fructose and the like, for example, a fructose-glucose syrup by a convenient method. Thus, the invention provides a solid-state saccharide composed of a plurality of sugars, that is, a composite crystal saccharide and/or granular crystals having D-fructose incorporated in the assembly of D-glucose monohydrate crystals; and a method of producing them. The invention contributes to expansion of the uses of the fructose-glucose syrup. In other words, the invention described in Patent Document 4 is an epoch-making one, breaking through the technical matters which have been considered as a common sense in the industry. The rare sugar-containing fructose-glucose syrup obtained by the above method is now used on trial as a sweetening agent, anti-obesity agent, appetite suppressant, insulin-resistance improver, or low-calorie sweetener and has attracting attentions as a saccharide having functionality.

The technology described in Patent Document 4 can be used for the composition of the present invention because it has a new composition composed of D-glucose, D-fructose, and D-psicose.

### [Comparison with conventional method in composition ratio]

According to one aspect of the production method of the present invention, a rare sugar-containing composition containing D-glucose, D-fructose, and D-psicose at a ratio of about 44:36:20 can be obtained.

In the production of a sweetener described in Patent Document 1, on the other hand, a sugar solution obtained by the method (1) using a continuous plant is a rare sugar-containing composition having D-glucose, D-fructose, and D-psicose at a ratio of 58:34:8 and it can be obtained by allowing, as ketohexose 3-epimerase, tagatose 3-epimerase to act on a fructose-glucose syrup which is generally and conventionally used one and is composed of 42 parts by weight of D-fructose and 58 parts by weight of glucose. A sugar solution obtained by the method (2) using a mixed enzyme is a rare sugar-containing composition having D-glucose, D-fructose, and D-psicose at a ratio of 41:48:11 obtained by 2-hour reaction or having D-glucose, D-fructose, and D-psicose at a ratio of 43:48:9 obtained by 1-hour reaction, each obtained by allowing a mixed enzyme of glucose isomerase and tagatose 3-epimerase to act on D-glucose. A sugar solution obtained by the method (3) of preparing a high D-psicose-containing sugar solution is a rare sugar-containing composition having D-glucose, D-fructose, and D-psicose at a ratio of 54:36:10 obtained by 4-hour reaction or having D-glucose, D-fructose, and D-psicose at a ratio of 41:42:17 by 20-hour reaction.

It has been found that compared with a conventional fructose glucose syrup or high fructose corn syrup, any of the rare sugar-containing compositions obtained using the methods described in Patent Document 1 has sweetness intensity and sweetness quality closer to those of sugar. It is also described in this document that further addition of fructose and D-glucose to these rare sugar-containing compositions is useful for controlling the sweetness and taste quality thereof.

The rare sugar-containing composition having D-glucose, D-fructose, and D-psicose at a ratio of about 44:36:20 obtained according to the one aspect of the production method of the present invention has a calculated sweetness intensity of 1.05, suggesting that compared with any of the rare sugar-containing compositions obtained in the methods described in Patent Document 1, the composition of the present invention has a sweetness intensity closer to that of sugar.

The novel sweetener thus obtained can be used in combination with a sweetener such as sucrose, sugar alcohol, aspartame, or stevia, according to preference. Water-soluble dietary fibers having a low sweetness intensity (such as polydextrose, inulin, or indigestible dextrin) may be added as needed to impart the composition with body feeling.

The aspect of the sweetener of the present invention embraces a rare sugar-containing composition having D-glucose, D-fructose, and D-psicose at a ratio of about 44:36:20, but when the composition is used while making use of its sweetness intensity and sweetness quality, its content is not particularly limited. The content can be adjusted as needed, depending on the degree of intended function, using aspect, using amount, or the like.

The novel sweetener of the present invention having a taste quality equal to that of sugar contains D-psicose and has an obesity preventing effect. It can also be used in combination with another active ingredient for preventing life style related diseases.

### [Uses]

The rare sugar-containing composition of the present invention containing from 10 to 20 parts by weight, preferably from 15 to 20 parts by weight of rare sugar psicose and from 90 to 80 parts by weight, preferably from 85 to 80 parts by weight of D-glucose and D-fructose, each based on 100 parts by weight in total of rare sugar D-psicose, D-glucose, and D-fructose and having a taste quality equal to that of sugar is a composition having a novel composition composed of D-glucose, D-fructose, and D-psicose so that it is used for the uses described in Patent Document 4.

The composition of the present invention is characterized in that it is a product of raw material D-fructose and a product not subjected to a separating operation by simulated moving bed chromatography. It is also characterized in that it is a product of raw material D-fructose and is a syrup obtained by passing through an immobilized enzyme reaction column. Thus, it is a composition having a new composition composed of D-glucose, D-fructose, and D-psicose so that it is also used for the uses described in Patent Document 4. Glucose, fructose, and D-psicose each belong to food (certified as food) so that they are very safe and easy to handle as a food mixture. D-psicose (D-allulose) is contained in food materials in a trace amount and is highly safe. Due to development of a mass production technology of it, it has recently a high utility value also in cost. It has already been known to have 5,000 mg/kg or more as a result of an acute oral toxicity test.

Examples of the uses of a sugar composition containing D-psicose (D-allulose) include food, beverages, particularly functional foods (for obesity prevention), pharmaceuticals, cosmetics, feed, agricultural chemicals (plant growth regulators, plant defense elicitors, and the like) and industrial use. Sweeteners having a taste quality equal to that of sugar can be used for anything requiring a sweet taste such as food, health foods, foods for patient, food materials, health food materials, food materials for patient, food additives, health food additives, food additives for patient, beverages, health beverages, beverages for patient, drinking water, health drinking water, drinking water for patient, drugs, raw materials for pharmaceutical, feed, and feed for patient livestock and/or patient animals.

When the sweetener of the present invention having a taste quality equal to that of sugar is used for food, it may be provided as is or may be prepared in a form diluted in water or the like, in a form suspended in oil or the like, as a meal in milky liquid form, or in a carrier-added form generally used in the food industry. The beverage is provided as a non-alcoholic beverage or an alcoholic beverage. Examples of the non-alcoholic beverage include carbonated beverages, fruit juice beverages, non-carbonated beverages such as nectar beverages, soft drinks, sports drinks, tea, coffee, and cocoa. Examples of the form of alcoholic beverage include beer, low-malt beer-like beverages, malt-free beer-like alcoholic beverages, sake, plum wine, wine, champagne, liqueur, white liquor highball, and medicinal alcoholic beverages.

When the rare sugar-containing composition of the present invention is used as a food material or food additive used for the purpose of improving the abnormal sugar metabolism and/or abnormal lipid metabolism, it is provided as tablets, capsules, powdery or granular solid agents to be dissolved in beverage or the like, semi-solids such as jelly, liquids such as drinking water, and highly-concentrated solutions to be diluted upon use.

Further, the rare sugar-containing composition of the present invention can be added to food as needed to obtain a health food or a food for the sick for the purpose of improving abnormal sugar metabolism and/or abnormal lipid metabolism. As an optional ingredient, vitamins, saccharides, coloring matters, flavoring agents, and the like that are usually added to food may be blended as needed. The food can be taken in any liquid or solid form. It can be taken in the form of a soft capsule obtained by encapsulating with gelatin or the like. The capsule is made of a gelatin film prepared by adding water to raw material gelatin to dissolve it and adding a plasticizer (glycerin, D-sorbitol, etc.) to the resulting solution.

The rare sugar-containing composition of the present invention can be used as a sweetener for the same uses of sugar. It can also be used for cooking, tea, coffee, seasonings (such as mirin), or the like.

The following are specific examples of food and beverages. Examples include western-type confectionary (pudding, jelly, gummy candy, candy, drop, caramel, chewing gum, chocolate, pastry, butter cream, custard cream, cream puff, pancake, bread, potato chip, fried potato, popcorn, cracker, pie, sponge cake, castella cake, waffle, cake, doughnut, biscuit, cookie, rice cracker, "okaki" cracker, "okoshi", sweet bun, candy etc.), dried noodle products (macaroni, pasta), egg products (mayonnaise, fresh cream), beverages (functional beverage, fermented lactic beverage, lactic acid bacteria beverage, concentrated dairy beverage, fruit juice beverage, fruit juice-free beverage, fruit pulp beverage, clear carbonated beverage, carbonated beverage with fruit juice, colored carbonated beverage with fruit), luxury products (green tea, black tea, instant coffee, cocoa, canned coffee drink), dairy products (ice cream, yogurt, coffee milk, butter, butter sauce, cheese, fermented milk, processed milk), pastes (marmalade, jam, flower paste, peanut paste, fruit paste, fruit preserved in syrup), livestock meat products (ham, sausage, bacon, dry sausage, beef jerky, lard), seafood products (fish ham, fish sausage, boiled fish-paste, tube-shaped fish paste cake, cake of ground fish combined with starch and steamed, dried fish, dried-bonito, dried-mackerel, dried boiled fish, sea urchin egg, fermented squid product, dried squid, fish dried with mirin, dried shellfish, smoked products such as smoked salmon), preserved food boiled in soy (small fish, shellfish, wild vegetable, mushrooms, kelp), curry (instant curry, retort-pouch curry, canned curry), seasonings (miso, powdered miso, soy sauce, powdered soy sauce, unrefined sake, fish sauce, sauce, ketchup, oyster sauce, solid bouillon, sauce for grilled meat, curry roux, stew mix, soup mix, instant bouillon, paste, instant soup, seasoned powder for sprinkling, dressing, salad oil), fried products (deep fried bean curd, fried confectionery, instant Chinese noodles), soy milk, margarine, and shortening.

The above-described food and beverages can be produced by blending the composition with raw materials of general food and processing by the conventional method. Although the blending amount of the composition in the food and beverages varies depending on the form of the food and is not particularly limited, it is usually preferably from 0.1 to 50 wt%.

The above-described food and beverages can also be used as functional foods, nutritional supplementary foods, or health foods. The form is not particularly limited and production examples of food include highly nutritional proteins with good amino acid balance such as milk proteins, soy protein, and egg albumin, degradation products thereof, oligopeptide of albumen, and soybean hydrolysate and also mixtures of simple amino acids. They can be used by the conventional method. They can also be used in the form of a soft capsule, a tablet, or the like.

Examples of the nutritional supplementary foods or functional foods include processed forms such as liquid foods, semi-digested nutritional foods, ingredient nutritional foods, health drinks, capsules, and enteral nutrients, each containing sugars, fats, trace elements, vitamins, emulsifiers, and flavoring agents. The above-described various foods, for example, food and beverages such as sports drinks and nutritional drinks may be supplemented further with nutritional additives such as amino acids, vitamins and minerals, sweeteners, spices, flavoring agents, and coloring matters to improve nutritional balance and flavor.

The functional foods are suited for use in the field of health foods or preventive medicines for preventing a specific disease (obesity prevention). The health foods for preventing a specific disease may contain as needed, in addition to a sugar composition containing rare sugar D-psicose (D-allulose) as an essential ingredient, an optional ingredient such as vitamins, saccharides, coloring matters, flavoring agents, and the like which have been added usually to food. The food can be taken in any liquid or solid form. It can be taken in the form of a soft capsule obtained by encapsulating with gelatin or the like. The capsule is made of a gelatin film prepared by adding water to raw material gelatin to dissolve it and adding a plasticizer (glycerin, D-sorbitol, etc.) to the resulting solution.

When the composition is used as a food additive, its form can be selected as needed. Examples include the sugar composition of the present invention containing rare sugar D-psicose (D-allulose) prepared as food as is, the composition added to another food, and optional forms such as capsules and tablets conventionally used for food or health foods. When the composition is taken or administered in a form blended in food, it may be mixed with an excipient, extender, binder, thickener, emulsifier, coloring matter, flavoring agent, food additive, seasoning, or the like as needed and the resulting mixture may be formed into powders, granules, tablets, or the like, depending on the intended use. Further, the composition is mixed in a food material to prepare a food and the food then industrialized as a functional food can be taken.

The composition of the present invention can be applied to feed for livestock, poultry and pets. For example, it can be blended in dry dog food, dry cat food, wet dog food, wet cat food, semi-moist dog food, poultry feed, and feed for livestock such as cattle and pigs. The feed itself can be prepared by the conventional method.

These therapeutic agents and preventives can also be used for non-human animals, for example, domestic mammals such as cattle, horses, pigs, and sheep, poultry such as chickens, quails, and, ostriches, reptiles, birds and pets such as small mammals, and even for farmed fish.

Drugs added for the purpose of expressing physiological action and displaying an effect of improving saccharide metabolism abnormalities and/or lipid metabolism abnormalities or an effect of improving obesity, while making use of the sweetness of the sweetener of the present invention may be used alone. Alternatively, after adding an appropriate additive such as general excipient, stabilizer, preservative, binder, or disintegrant and forming the resulting mixture into an appropriately selected form such as liquids, granules, fine granules, powders, tablets, capsules, pills, sprays, or spraying agents, they can be orally or nasally administered.

For preparing the composition of the present invention as a drug, usable are pharmaceutical organic or inorganic solid, semi-solid or liquid carriers, solubilizers or diluents suitable for oral or nasal administration. Any of water, gelatin, lactose, starch, magnesium stearate, talc, animal·vegetable oil, benzyl alcohol, gum, polyalkylene glycol, petroleum resin, coconut oil, lanolin, and other carriers (carriers) can be used as a carrier of a drug containing the composition of the present invention. Further, stabilizers, wetting agents, emulsifiers, and salts for changing the osmotic pressure or maintaining an appropriate pH of an agent to be blended can be used as needed as an adjuvant.

When the sugar composition is used as a pharmaceutical composition, a known method can be used for the preparation thereof. The dosage form can be selected as needed. As such a dosage form, for example, when it is obtained as an orally administrable preparation, examples of the dosage form include tablets, granules, powders, capsules, coating agents, liquids, and suspensions. When it is obtained as a parenterally administrable administration, examples of the form include injections, drops, and suppositories. The dose of the pharmaceutical composition can be set as needed, depending on its form, administration route, and using purpose of the pharmaceutical composition and the age, weight, and symptom of a subject to be administered. In the drug, a sugar composition containing rare sugar D-psicose (D-allulose) as an active ingredient can be used not only as is but also as a pharmaceutically acceptable salt thereof. As the drug, the sugar composition containing D-psicose (D-allulose) can be used singly as a preparation and in addition, a preparation composition obtained by adding a pharmaceutically usable carrier or diluent and processing the mixture can be used. Such a preparation or drug composition can be administered, as described above, through an oral route or a parenteral route. For example, a solid or fluid (gel and liquid) preparation or drug composition for oral administration is provided in the form prepared as tablets, capsules, tablets, pills, powders, granules, or gels. An accurate dose of the preparation or drug composition changes with the intended use form or treatment time so that it is an adequate amount determined by a doctor or veterinarian in charge. A dose or administered amount can be adjusted as needed by the preparation form. A daily dose of an oral solid preparation such as tablets or that of an oral liquid may be administered once or in several portions. In a preparation form such as syrup, troches, or chewable tablets which infants take to cause a local action and also cause a systemic action by oral administration, 1/2 to 1/10 of a daily dose may be blended and administered as a single dose and in this case, a total dose does not necessarily satisfy a daily dose.

On the contrary, if an administration amount is reasonable judging from the preparation form, an amount corresponding to a daily dose may be blended for a single dose. In obtaining a preparation, ordinarily employed fillers, extenders, binders, disintegrants, surfactants, lubricants, coating agents, sustained release agents, diluents, and excipients can be used. In addition, usable are solubilizing agents, buffers, preservatives, solubilizers, isotonizing agents, emulsifying agents, suspending agents, dispersants, thickeners, gelling agents, curing agents, absorbents, adhesives, elasticity imparting agents, plasticizers, adsorbents, flavoring agents, coloring agents, taste corrigents, antioxidants, moisturizing agents, light shielding agents, brighteners, antistatic agents, and the like.

The present invention can provide a skin external preparation making use of the skin moisturizing, skin antioxidant, and skin antiaging effects of rare sugar D-psicose (D-allulose), that is, a skin external preparation which has an improving·preventive effect against skin roughness or dry or chapped skin and is known as a remedy, a skin external preparation, a cosmetic, or the like (Patent Document 7). The skin external preparation according to the present invention may contain, in addition to a sugar composition containing rare sugar D-psicose (D-allulose) as an essential ingredient, ingredients ordinarily used for skin external preparations such as cosmetics and pharmaceuticals, for example, water-based ingredients, oily ingredients, powdery ingredients, alcohols, moisturizing agents, thickeners, ultraviolet absorbers, whitening agents, antiseptics, antioxidants, surfactants, perfumes, coloring agents, and various skin nutrition agents as needed, if necessary Further, skin external preparations may contain metal sequestering agents such as disodium edetate, trisodium edetate, sodium citrate, sodium polyphosphate, sodium metaphosphate, and gluconic acid, hot water extracts such as caffeine, tannin, verapamil, herbal extracts, glabridin, and Chinese quince, various crude drugs, drugs such as tocopherol acetate, glycyrrhizic acid, and tranexamic acid and derivatives thereof, or salts thereof, Vitamin C, magnesium ascorbate phosphate, glucoside ascorbate, albutin, kojic acid, and sugars such as D-glucose, D-fructose, and trehalose. This means that a skin external preparation can be produced by blending, in a sugar composition containing rare sugar D-psicose (D-allulose), a raw material ordinarily used for skin external preparation, for example, an oil or fat such as vegetable oil, a wax such as lanolin or beeswax, a hydrocarbon, a fatty acid, a higher alcohol, an ester, a surfactant, a coloring matter, a perfume, a vitamin, an extracted ingredient from vegetable or animal, ultraviolet absorber, antioxidant, antiseptic, or bactericide. A herbal extract ingredient such as glycyrrhetinic acid, diphenhydramine hydrochloride, azulene, dl-α-tocopherol and derivatives thereof, vitamin B2, vitamin B6, and the like which are raw materials for anti-inflammatory skin external preparations may be used in combination. The skin external preparation may be provided in any form such as ointment, cream, emulsion, lotion, pack, or bath additive insofar as it is conventionally used for skin external preparations and the dosage form is not particularly limited. When the composition is used as a skin external preparation, the dosage form can be selected as needed. Examples include a solution system, a solubilized system, an emulsified system, a powder dispersion system, a water-oil two-layer system, a water-oil-powder three-layer system, gel, mist, spray, mousse, roll-on, and stick and further, a preparation obtained by impregnating a sheet such as nonwoven cloth with the composition or applying the composition thereto.

With a view to providing a material having possibility of remarkably reducing the using amount of an agricultural chemical and having amplifying action of plant disease resistance, another group of the present inventors have already registered an invention of a plant disease resistance amplifying agent containing a rare sugar (Patent Document 8). The research and development of it have proceeded rapidly and there is a plurality of patents on it.

The composition can be used as a sweetener to be added to toothpaste.

Further, a soluble film has been used for the formulation of cosmetics and the like. For example, an edible soluble film is used as a flavor film or the like having a flavoring agent or the like retained thereon for the purpose of mood change, prevention of bad breath, or the like. There is proposed a soluble film which exhibits excellent solubility and film properties as a packing material of food, pharmaceuticals, and the like or as a carrier for retaining an active ingredient of food, pharmaceuticals, or the like and is suitably used for these applications (Patent Document 9). Thus, the sweetener of the present invention having a sweetness intensity and sweet taste equal to those of sugar can be applied to pharmaceuticals, quasi drugs, and cosmetics.

The blending amount in food, beverage, cosmetic, or feed is not particularly limited, but D-psicose (D-allulose) is blended preferably in an amount of from 0.01 to 10 wt%. In the case of a pharmaceutical, it can be orally administered as a capsule, powder, or tablet. Since it is soluble in water, an administration route such as intravenous injection or intramuscular injection may be used as well as oral administration. The dose differs depending on the degree of the symptom of, for example, diabetes, weight, age, sex, or the like. Upon use, it is desired to determine an appropriate dose depending on the symptom. The blending amount in a pharmaceutical is not particularly limited, but from about 0.01 to 2,000 mg of D-psicose (D-allulose) is preferred for oral administration, from about 0.01 to 1,000 mg for intravenous injection administration, and from about 0.01 to 1,000 mg for intravenous injection administration, each per kg of weight.

The present invention will be described in further detail by Examples, but the present invention is not limited to or by Examples.

### Example 1

The production of rare sugar by using isomerase usually includes the following four steps:
(1) culturing of a microorganism and mass production of an enzyme,
(2) enzymatic reaction to convert into a rare sugar,
(3) separation of the rare sugar as a product from a raw material (such as fructose) when the reaction is equilibrium one, and
(4) concentration of the rare sugar. If necessary, the following fifth step:
(5) crystallization of the rare sugar is added. These steps are carried out efficiently to achieve mass production of the rare sugar.

One example of a preliminary test is shown in the present example.

The production method in the preliminary test is characterized in that the separation (3) of the rare sugar from a raw material (such as fructose) is not performed.

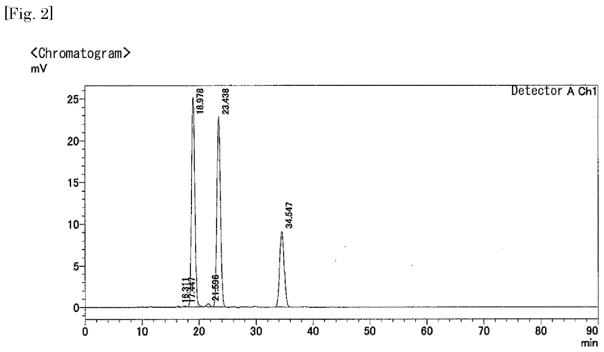

As shown in Fig. 1, in a conventional step of preparing D-psicose from D-fructose by allowing ketose 3-epimerase to act on raw material D-fructose to convert D-fructose into D-psicose in Reaction 1, a D-xylose isomerase reaction of Reaction 2 was performed without separating D-psicose. The reaction product which was eluting from a column over time was sampled and analyzed by HPLC (detector: RI, column: MCI GEL CK 08EC, product of Mitsubishi Chemical, mobile phase: water, flow rate: 0.4 ml/min) (the analysis conditions will hereinafter be the same in the following test example). HPLC analysis results are shown in Fig. 2. The sugar solution obtained as the product of Reaction 2 was found to be roughly composed of 43 parts by weight of D-glucose, 37 parts by weight of D-fructose, and 20 parts by weight of D-psicose.

The rare sugar-containing composition obtained by the production method of the present invention has a sweetness intensity closer to that of sugar, which will be described in detail hereinafter.

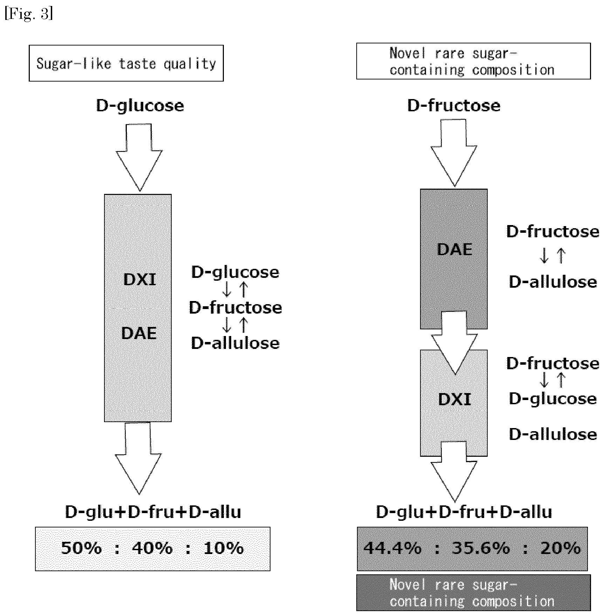

The production step shown on the left side of Fig. 3 is a step for producing a sweetener having a sweetness intensity and a taste quality equal to those of sugar and it is described in

Patent Document 1. It shows that the mixed sugar composition thus obtained contains D-glucose, D-fructose, and D-psicose at a ratio of 50:40:10.

On the other hand, the step of producing a novel rare sugar-containing composition of the present invention (production step of the above preliminary test) in Fig. 2 is shown on the right side of Fig. 3. These two production steps are shown in contrast. The product of the preliminary test produced by the production step shown on the right side of Fig. 3 is a mixed sugar composition containing D-glucose, D-fructose, and D-psicose at a ratio of 44.4:30.6:20.

The sweetness and sweet taste quality of these two mixed sugar compositions will next be described.

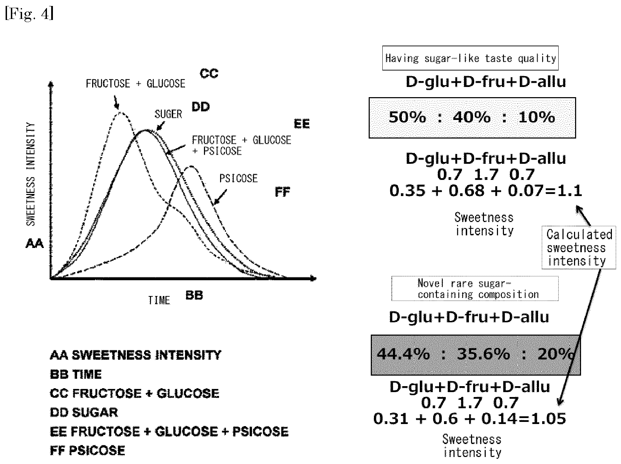

A graph of a sweetness curve is shown on the left side of Fig. 4. In this graph, Fig. 2 of Patent Document 1 is cited. In this graph, AA means sweetness intensity, BB means time, CC means D-glucose + D-fructose, DD means sugar, EE means D-fructose + D-glucose + D-psicose, and FF means D-psicose. As CC, a high fructose corn syrup (55 parts by weight of fructose + 45 parts by weight of glucose) is used.

D-glucose has about 70% of the sweetness intensity of sugar and D-fructose has about 170% of the sweetness intensity of sugar. This is the reason why D-fructose is said to be sweet and delicious. The sweetness intensity and taste quality of a fructose-glucose syrup or high fructose corn syrup differ, depending on the respective content ratios of D-fructose and D-glucose. It is known that compared with a sweetener composed of a single ingredient, a mixed sugar generally has a wide sweetness peak width and shows mild sweetness properties. It has been found from the organoleptic test results (refer to Table 1) of Example 2 in Patent Document 1 that a composition containing glucose and psicose at a ratio of about 9:1 has a sweetness intensity and a sweetness quality close to those of sugar. This document has also revealed that a combination having a sweetness intensity and/or a taste quality close to those of sugar is a mixed sugar containing fructose in an amount within a range of from 30 parts by weight to 80 parts by weight and containing D-glucose and D-psicose in an amount within a range of (from 70 parts by weight to 20 parts by weight).

Fructose-glucose syrups are frequently used for soft drinks or frozen dessert because their sweetness intensity increases under low temperatures. Fructose has an effect of encouraging the flavor of fruit (particularly, citrus fruit) so that it is used for canned fruit or fruit beverages. Attention has been paid to the relationship for these 20 to 30 years between the caloric intake of D-fructose (fructose) constituting a fructose-glucose syrup and an increase in the number of obese people and patients suffering from life style related diseases. Although the absorption rate of fructose, which is a monosaccharide, from the intestinal tract is slow, a metabolic rate in the liver is considered to be high. Excessive intake of fructose has so far been believed to accelerate synthesis·secretion of neutral fat and become a cause of obesity or hyperlipidemia. In experimental animal rats who have taken a high fructose diet, fructose inhibits the reaction of insulin, increases a uric acid level in the blood, and enhances appearance of metabolic syndrome such as hypercholesterolemia or hypertension. The reason why a fructose-glucose syrup or high fructose corn syrup is a main cause of obesity is because D-fructose (fructose) has an adverse effect on the accumulation of neutral fat or the like. Excessive intake of fructose certainly damages one's health, but it is presumed that fructose stabilizes a blood glucose level and is not hard on the body. It is known that an adequate intake amount of fructose promotes the utilization of fat, controls degradation of liver glycogen, and enhances endurance in exercise. There is a demand for the improvement of fructose-glucose syrup as a sweetener by finding an appropriate intake amount of fructose that does not damage the body and is useful for health and achieving this intake amount. The present invention has succeeded in satisfying the adequate intake amount of D-fructose (fructose) with a view to improving the fructose-glucose syrup by paying attention to the functionality of D-allulose (D-psicose). More specifically, the present invention has succeeded in adjusting the amount of D-fructose so as to prevent appearance of its defect, controlling the composition to bring out an advantage that D-fructose is sweet, making up for the insufficient sweetness intensity which is a defect of D-psicose, and thus creating a new sweetener having functionality of D-psicose.

In the mixed sugar composition of the present invention having D-glucose, D-fructose, and D-psicose at a ratio of 44.4:30.6:20, the amount of fructose, 30.6 parts by weight, is an amount close to the lower limit in the amount range of the fructose from 30 parts by weight to 80 parts by weight (Patent Document 1). It is an amount adjusted so as to prevent appearance of the defect (adverse effect on accumulation of neutral fat or the like) of D-fructose which is sweet and delicious but an amount enough for making up for the insufficient sweetness intensity of D-psicose which is a disadvantage thereof.

The amount of D-glucose and D-psicose, 64.4 parts by weight, is an amount close to the upper limit in the amount range of D-glucose and D-psicose from 70 parts by weight to 20 parts by weight (Patent Document 1) and this amount makes it possible to create a sweetener which contains D-psicose in an amount bringing out the functionality of D-psicose and has a sweetness balance among D-glucose, D-fructose, and D-psicose close to that of sugar.

In addition, D-psicose has such properties that it presents a refreshing and cool taste but provides sweetness a littler later. The sweetness intensity of D-psicose is about 70% of that of sugar and when it is used alone as a sweetener, it is different from sugar in sweetness intensity and sweetness quality.

It can be understood from these facts that by the addition of D-psicose, a sweetness curve shown in Fig. 4 changes from Curve CC of a high fructose corn syrup (55 parts by weight of fructose + 45 parts by weight of glucose) to Curve EE of a mixed sugar curve (fructose + glucose + psicose), suggesting that the taste quality becomes closer to that of Sugar DD.

On the right side of Fig. 4, the sweetness intensity of a mixed composition containing D-glucose, D-fructose, and D-psicose at a ratio of 50:40:10 is shown on the upper side and the sweetness intensity of the product of the preliminary test in Example 1 containing D-glucose, D-fructose, and D-psicose at a ratio of 44.4:30.6:20 is shown on the lower side, each as a result of calculation. This has revealed that the mixed composition of D-glucose, D-fructose, and D-psicose prepared in Example 1 shows a taste essentially identical to that of sugar.

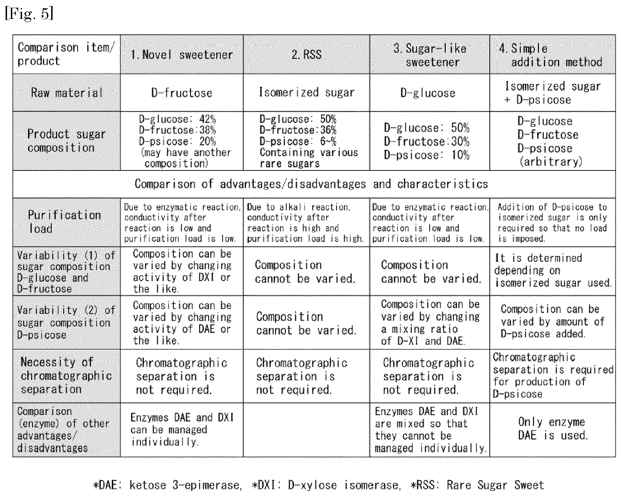

Characteristics (comparison items: details of product, standard of contamination level, adjustment of reaction rate, use and disuse of chromatographic fraction collector, exchange of immobilized enzyme) of the method (preliminary test) of producing a rare sugar composition having a taste quality equal to that of sugar by using D-fructose as a raw material will be described referring to Fig. 5 in contrast with the production method described in Patent Document 5, the production method described in Patent Document 1, and a simple addition method.

The term "novel sweetener", "RSS", "sugar-like sweetener", and "simple addition method" used in Fig. 5 are each a rare sugar-containing composition as described below.
1. Novel sweetener: the method of the present invention for producing a rare sugar composition having a taste quality equal to that of sugar by using D-fructose as a raw material.
2. RSS: "Rare Sugar Sweet" (product of Matsutani Chemical Industry), a rare sugar syrup (trade name: Rare Sugar Sweet) produced by the production method described in Patent Document 5. A rare sugar-containing sugar composition containing from 0.5 to 17 mass% of D-psicose, from 0.2 to 10 mass% D-allose, and other rare sugars.
3. Sugar-like sweetener: a novel sweetener produced by the production method described in Patent Document 1 and having a sugar-like taste quality.
4. Simple addition method: a mixture of D-glucose, D-fructose, and D-psicose simple product. For the production of a simple product, a problem of purification (such as crystallization) should be overcome.

### Example 2

This example shows that a D-glucose:D-fructose:D-psicose ratio can be changed to suit the purpose by changing the activity (enzyme level) of D-xylose isomerase in the second reaction.

Another important characteristic of the present invention is the possibility of preparing a product not having a predetermined composition but having a composition changed according to the purpose. It has been confirmed that products having various compositions can be obtained by changing the reaction conditions. Products having different compositions can be produced by changing the enzymatic activity (immobilized enzyme level) of both enzymes or the passage rate through an immobilized enzyme column. The present example shows the possibility caused by a change in enzyme level.

The following is a study of characteristic of the present invention made by changing the activity (enzyme level) of D-xylose isomerase in the second reaction and analyzing the composition of the product.

First, an equilibrium mixture of D-fructose and D-psicose was produced by allowing ketose 3-epimerase to act on D-fructose.

Cells obtained by adding 2% D-psicose to an inorganic salt medium and culturing Arthrobacter globiformis M30 on the resulting medium were suspended in a Mg₂SO₄ solution (same amount as the weight of the cells) having a concentration of 50 mM. The enzyme was extracted and subjected to a high-speed centrifuge to obtain a supernatant (enzyme solution: D-allulose 3-epimerase [DAE] crude enzyme solution).

The Arthrobacter globiformis M30 strain is internationally deposited as deposit number NITE BP-1111 in the National Institute of Technology and Evaluation, Patent Microorganisms Depositary Center (2-5-8, Kazusa Kamatari, Kisarazu, Chiba, Japan) (Patent Document 6).

An ion exchange resin (anionic resin) was put in the resulting enzyme solution and the resulting mixture was stirred at 5°C for 40 hours to obtain an immobilized enzyme. The DAE immobilized enzyme thus obtained was packed in a 100 ml immobilization reaction column.

A solution obtained by adding 20 mM Mg₂SO₄ to a D-fructose solution having a concentration of 50% and adjusting the resulting mixture to pH 7.5 with a 0.5 M NaOH solution was passed through a DAE-immobilized enzyme column at a flow rate of 100 ml/hour. After the passage, the solution was analyzed by HPLC to have 79% D-fructose and 20% D-psicose.

The composition of the product was investigated by using the mixture of D-fructose and D-psicose thus obtained and changing an immobilized enzyme level of D-xylose isomerase in the second reaction.

### [Production 1]

80 ml of a commercially-available D-xylose isomerase immobilized enzyme (product of Novo) was dispersed in 100 ml of a 150 mM
Mg₂SO₄ solution and after sufficient hydration, the resulting dispersion was packed in a 100 ml immobilization reaction column. Then, the solution obtained by the reaction of the DAE immobilized enzyme was passed through the D-xylose isomerase immobilized enzyme column at a flow rate of 100 ml/hour.

A reaction solution obtained by passing through the column packed with 80 ml of D-xylose isomerase was analyzed by HPLC to find that it contained 40% D-glucose, 39% D-fructose, and 20% D-psicose.

### [Production 2]

Similarly, the solution (79% D-fructose : 20% D-psicose) obtained by DAE reaction was passed through an immobilized enzyme column with 50 ml of D-xylose isomerase at a flow rate of 100 ml/hour.

A solution obtained by passing through the immobilized enzyme column with 50 ml of D-xylose isomerase was analyzed by HPLC to find that it contained 25% D-glucose, 54% D-fructose, and 20% D-psicose.

### [Production 3]

Similarly, the solution (79% D-fructose : 20% D-psicose) obtained by DAE reaction was passed through an immobilized enzyme column with 20 ml of D-xylose isomerase at a flow rate of 100 ml/hour.

A solution obtained by passing through the immobilized enzyme column with 20 ml of D-xylose isomerase was analyzed by HPLC to find that it contained 18% D-glucose, 61% D-fructose, and 20% D-psicose.

### Example 3

This example shows that a D-glucose:D-fructose:D-psicose ratio can be changed to fit the purpose by changing an enzyme level of ketose 3-epimerase in the first reaction.

Investigation was made by using, as a substrate, a product of D-fructose and D-psicose obtained by changing the enzyme level of ketose 3-epimerase in the first reaction.

The test was performed by obtaining mixed solutions different in the composition of D-fructose and D-psicose by using the ketose 3-epimerase obtained in Example 2 and decreasing the enzyme level to 20 ml from 50 ml. The test of changing an enzyme level was performed using the resulting mixed solutions for the second D-xylose isomerase reaction.

A solution obtained by packing 20 ml of an immobilized DAE enzyme in an immobilization reaction column and a solution obtained by adding 20 mM Mg₂SO₄ to a D-fructose solution having a concentration of 50% and adjusting the resulting mixture to pH 7.5 with a 0.5 M NaOH solution was passed at a flow rate of 100 ml/hour. The solution after the passage was analyzed by HPLC to find to have 85% D-fructose and 15% D-psicose. The solution was used for the subsequent test as a reaction substrate.

By using the resulting solution having 85% D-fructose and 15% D-psicose, the composition of the product was analyzed by changing the activity (enzyme level) of D-xylose isomerase in the second reaction. The test is similar to that in Example 2 performed by changing the level of D-xylose isomerase.

### [Production 1]

The solution having 85% D-fructose and 15% D-psicose was passed through 100 ml of immobilized D-xylose isomerase at a flow rate of 100 ml/hour. The solution after the passage was analyzed by HPLC to find that it contained 43% D-glucose, 41% D-fructose, and 15% D-psicose.

### [Production 2]

The solution having 85% D-fructose and 15% D-psicose was passed through 50 ml of immobilized D-xylose isomerase at a flow rate of 100 ml/hour. The solution after the passage was analyzed by HPLC to find that it contained 27% D-glucose, 51% D-fructose, and 15% D-psicose.

### [Production 3]

The solution having 85% D-fructose and 15% D-psicose was passed through 20 ml of immobilized D-xylose isomerase at a flow rate of 100 ml/hour. The solution after the passage was analyzed by HPLC to find that it contained 19% D-glucose, 59% D-fructose, and 15% D-psicose.

The above-described results of Example 2 and Example 3 have shown that the D-glucose:D-fructose:D-psicose ratio of the product can be changed depending on the purpose by changing the enzyme level of the enzymes D-xylose isomerase and ketose 3-epimerase to be used.

The finding that the ratio can be changed with a change in the enzyme level has also shown that a similar product can be obtained even by changing the flow rate of the solution through a column.

These results show that a D-furctose:D-psicose ratio can be controlled within a range of from 99:1 to 80:20; a D-glucose:D-fructose ratio can be controlled within a range of from 1:99 to 41:49; and an overall D-psicose ratio can be controlled by the first reaction and a D-glucose:D-fructose ratio can be controlled by the second reaction.

For example, it is recommendable to select D-fructose abundant conditions to obtain a sweet product and to set the amount of D-psicose at the maximum ratio to allow D-psicose to display its functionality. Thus, as a production method of a new sweetener, the technology developed herein has widened the possibility.

### Industrial Applicability

A sweetener having a sweetness intensity and a taste quality almost equal to that of sugar but not causing life related diseases such as obesity though used widely in the food industry and the like is expected to be mass produced at low cost. Success in mass production is expected to expand the uses of a sugar composition containing D-psicose (D-allulose) to food, beverages, particularly functional foods (for obesity prevention and the like), pharmaceuticals, cosmetics, feed, agricultural chemicals (plant growth regulators, plant defense elicitors, and the like), and industrial use.

## Claims

1. A method of producing a rare sugar-containing composition with D-fructose as a raw material, comprising a first stage of preparing a mixture of D-fructose and D-psicose by using ketose 3-epimerase and a second stage of converting D-fructose into D-glucose by using D-xylose isomerase inert to D-psicose to obtain an intended product having a new composition of D-glucose, D-fructose, and D-psicose.

2. The method according to Claim 1, wherein the first-stage product is a mixture of D-fructose and rare sugar D-psicose.

3. The method according to Claim 1, wherein the first-stage product is an equilibrium mixture of D-fructose and rare sugar D-psicose.

4. The method according to Claim 1, wherein the first-stage product is a mixture of D-fructose and rare sugar D-psicose adjusted to have an intended concentration.

5. The method according to any of Claims 1 to 4, wherein in the second stage, D-xylose isomerase is allowed to act on the first-stage product continuously or individually.

6. The method according to any of Claims 1 to 5, wherein the second-stage intended product is a composition having from 10 to 20 parts by weight of rare sugar D-psicose and from 90 to 80 parts by weight of D-glucose and D-fructose, each based on 100 parts by weight in total of rare sugar D-psicose, D-glucose, and D-fructose.

7. The method according to any of Claims 1 to 5, wherein the second-stage intended product is a composition having from 15 to 20 parts by weight of rare sugar D-psicose and from 85 to 80 parts by weight of D-glucose and D-fructose, each based on 100 parts by weight in total of rare sugar D-psicose, D-glucose, and D-fructose.

8. The method according to any of Claims 1 to 7, wherein the second-stage intended product is a rare sugar-containing composition having a taste quality equal to that of sugar.

9. The method according to any of Claims 1 to 8, wherein the intended product having an intended composition is obtained by adjusting the enzymatic activity of ketose 3-epimerase and D-xylose isomerase used.

10. The method according to any of Claims 1 to 9, wherein the reaction in the first stage and the reaction in the second stage are performed by passing substrate solutions through immobilized enzyme columns, respectively, and an intended product having an intended composition is obtained by adjusting a flow rate of substrates.

11. The method according to any of Claim 1 to Claim 10, wherein an immobilized enzyme reaction is performed with raw material D-fructose as a substrate and a 100% intended product is obtained theoretically without producing a by-product.

12. The method according to any of Claims 1 to 11, wherein the intended product is not lost during a production step by a separating operation using simulated moving bed chromatography.

13. The method according to any of Claims 1 to 12, wherein a saccharide in crystal form or solid form is prepared from a syrup itself obtained by passing the second-stage intended product through an immobilized enzyme column.

14. A rare sugar-containing composition having a taste quality equal to that of sugar, comprising from 10 to 20 parts by weight of rare sugar D-psicose and from 90 to 80 parts by weight of D-glucose and D-fructose, each based on 100 parts by weight in total of rare sugar D-psicose, D-glucose, and D-fructose.

15. A rare sugar-containing composition having a taste quality equal to that of sugar, comprising from 15 to 20 parts by weight of rare sugar D-psicose and from 85 to 80 parts by weight of D-glucose and D-fructose, each based on 100 parts by weight in total of rare sugar D-psicose, D-glucose, and D-fructose.

16. The rare sugar-containing composition having a taste quality equal to that of sugar according to Claim 14 or 15, which is a product of raw material D-fructose and is a product not subjected to a separating operation by simulated moving bed chromatography.

17. The rare sugar-containing composition having a taste quality equal to that of sugar according to Claim 14, 15, or 16, which is a product of raw material D-fructose and at the same time, is a syrup itself obtained by passing through the immobilized enzyme reaction column.

18. The rare sugar-containing composition having a taste quality equal to that of sugar according to any of Claims 14 to 17, which is a sweetener having a taste quality equal to that of sugar.

19. The rare sugar-containing composition having a taste quality equal to that of sugar according to any of Claims 14 to 18, which is in syrup form or in crystalline form.

20. A sugar-like sweetener comprising the rare sugar-containing composition having a taste quality equal to that of sugar as claimed in any of Claims 14 to 19.

21. A food comprising the rare sugar-containing composition having a taste quality equal to that of sugar as claimed in any of Claims 14 to 19.

22. A pharmaceutical or quasi drug comprising the rare sugar-containing composition having a taste quality equal to that of sugar as claimed in any of Claims 14 to 19.

23. An oral composition comprising the rare sugar-containing composition having a taste quality equal to that of sugar as claimed in any of Claims 14 to 19.

24. A cosmetic comprising the rare sugar-containing composition having a taste quality equal to that of sugar as claimed in any of Claims 14 to 19.

25. An anti-obesity agent comprising the rare sugar-containing composition having a taste quality equal to that of sugar as claimed in any of Claims 14 to 19.

26. An appetite suppressant comprising the rare sugar-containing composition having a taste quality equal to that of sugar as claimed in any of Claims 14 to 19.

27. An insulin-resistance improver comprising the rare sugar-containing composition having a taste quality equal to that of sugar as claimed in any of Claims 14 to 19.

28. A low-calorie sweetening agent comprising the rare sugar-containing composition having a taste quality equal to that of sugar as claimed in any of Claims 14 to 19.

29. A sugar-like sweetening agent comprising the rare sugar-containing composition having a taste quality equal to that of sugar as claimed in any of Claims 14 to 19.
